(19) Europäisches Patentamt
European Patent Office
Office européen des brevets

(11) Publication number: **0 133 934**
**A2**

(12) **EUROPEAN PATENT APPLICATION**

(21) Application number: **84108413.0**

(22) Date of filing: **17.07.84**

(51) Int. Cl.⁴: **C 07 D 513/14**, A 61 K 31/505
//
(C07D513/14, 333/00, 285/00, 239/00)

(30) Priority: **29.07.83 CH 4164/83**

(43) Date of publication of application: **13.03.85**
**Bulletin 85/11**

(84) Designated Contracting States: **AT BE CH DE FR GB IT LI LU NL SE**

(71) Applicant: **ETHACHEM S.A., Via Vela, 3, CH-6830 Chiasso (CH)**

(72) Inventor: **Canova, Arnaldo, Via Vela, 3, CH-6830 Chiasso (CH)**

(74) Representative: **Bianchetti, Giuseppe, Studio Consulenza Brevettuale Via Senato 24, I-20121 Milan (IT)**

(54) **Pharmacologically active 1,3,4-thiadiazol-(3,2-a)-thieno-(2,3-d)-pyrimidin-5-(H)one derivatives.**

(57) 1,3,4-Thiadiazol-[3,2-a]-thieno-[2,3-d]-pyrimidin-5-[H]one derivatives of formula (I)

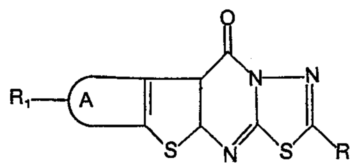

(I)

wherein R = H, alkyl, alkylmercapto, alkoxy, aryl; A = trimethylene, tetramethylene or $-CH=CH-CH=CH-$; $R_1$ = H, alkyl, alcoxy or hydroxy and a process for the preparation thereof.

Compounds (I) are endowed with interesting analgesic and antiinflammatory activities.

EP 0 133 934 A2

Pharmacologically active 1,3,4-thiadiazol-/3,2-a/-thieno-/2,3-d/-pyrimidin-5-/H/one derivatives

The present invention relates to 1,3,4-thiadiazol-/3,2-a/-thieno-/2,3-d/-pyrimidin-5-/H/one derivatives, having general formula

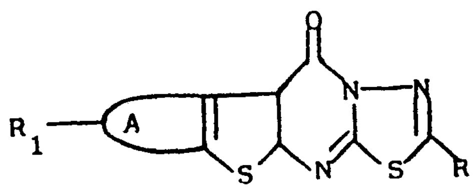

(I)

wherein R is hydrogen or one of the following groups: H, $CH_3$, $C_2H_5$, $SCH_3$, $SC_2H_5$, $OC_2H_5$, $C_6H_5$, $C_6H_4F(p)$, $C_6H_4OC_2H_5(o)$;
A is a tetramethylene, trimethylene or -CH=CH-CH=CH- group; $R_1$ is hydrogen, $C_1$-$C_4$ alkyl, $C_1$-$C_4$ alcoxy, hydroxy.

Compounds I have analgesic and antiinflammatory activities.

Therefore, another object of the invention relates to therapeutic compositions comprising as the active ingredient one or more compounds according to the invention.

The present invention relates also to a process for the production of compounds of formula I, which consists in reacting 2-amino-3-carbalcoxythiophenes (II) with 2-halo-1,3,4-thiadiazoles (III), according to the following reaction scheme:

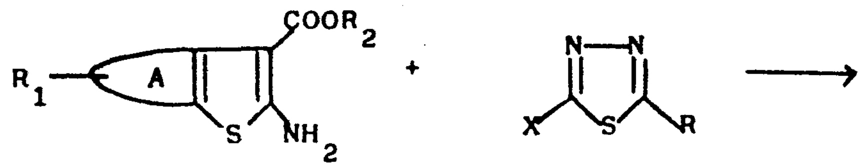

(II) (III) (I)

wherein A, R and $R_1$ have the above mentioned meanings; X is an halogen atom and $R_2$ is $C_1$-$C_4$ alkyl. The reaction is carried out in the presence or in absence of solvents, at temperatures ranging between 80 and 220°C, preferably from 120 to 180°C.

The following example illustrates the process according to the invention, without limiting it.

EXAMPLE

2-Methyl-mercapto-6,7,8,9-tetrahydro-5H/1/-benzothieno/2,3-d/-1,3,4-thiadiazole/3,2-a/pyrimidin-5/H/-one (code : HA 34)

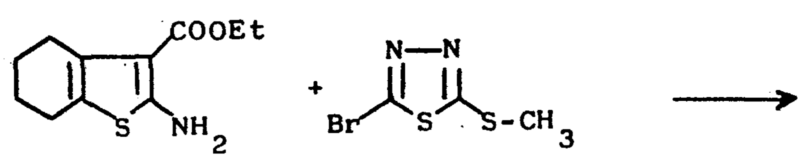

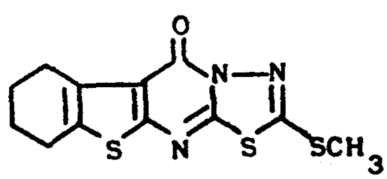

(H 34)

0.01 Moles of 2-amino-3-carbethoxy-4,5,6,7-tetrahydrobenzo-/1/-thiophen were added with 0.01 moles of 2-bromo-5-methylmercapto-1,3,4-thiadiazol, with stirring. The reaction mixture was slowly heated till 150°C; heating was continued for

half an hour at the same temperature. After cooling, the mixture was treated with ethanol (∿40 ml), heated to reflux and cooled. The resulting residue was filtered, treated with a sodium hydrogenocarbonate solution and crystallized from ethanol-dioxane, to give the title compound, in the form of needles, having melting point of 195°C. The structure of the obtained compound was in accordance with the analytical and spectroscopic data.

Repeating the procedure described in the Example, compounds I were obtained, which are shown in the following Table I. The spectroscopic data (IR, UV, NMR) and the elemental analysis were in accordance with the expected structures.

TABLE I

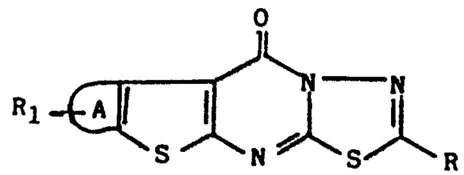

(I)

| Code | A | R | R1 | Elemental formula | M.W. | M.P. |
|---|---|---|---|---|---|---|
| HA 30 | $-(CH_2)_3-$ | $CH_3$ | H | $C_{11}H_{10}N_3S_2O$ | 264.34 | 196°C |
| HA 31 | -CH=CH-CH=CH- | $CH_3$ | $C_2H_5$(6) | $C_{14}H_{12}N_3S_2O$ | 302.39 | 221°C |
| HA 32 | $-(CH_2)_4-$ | $C_2H_5$ | H | $C_{13}H_{13}N_3S_2O$ | 291.38 | 186°C |
| HA 33 | $-(CH_2)_4-$ | $C_6H_5$ | H | $C_{17}H_{13}N_3S_2O$ | 339.42 | 195°C |
| HA 34 | $-(CH_2)_4-$ | $SCH_3$ | H | $C_{12}H_{11}N_3S_3O$ | 309.42 | 255°C |
| HA 35 | -CH=CH-CH=CH- | $SC_2H_5$ | $C_2H_5$(6) | $C_{15}H_{13}N_3S_3O$ | 347.46 | 243°C |
| HA 36 | $-(CH_2)_4-$ | $C_6H_4OC_2H_5$(o) | H | $C_{19}H_{17}N_3S_2O_2$ | 383.47 | 274°C |
| HA 37 | $-(CH_2)_3-$ | $OC_2H_5$ | $OC_2H_5$(6) | $C_{14}H_{15}N_2S_2O_3$ | 323.40 | 224°C |
| HA 38 | $-(CH_2)_4-$ | $C_6H_4F$(p) | $OC_2H_5$(6) | $C_{19}H_{16}N_2S_2O_2F$ | 387.46 | 199°C |
| HA 39 | $-(CH_2)_3-$ | $C_6H_4OC_2H_5$(o) | OH(6) | $C_{18}H_{15}N_2S_2O_3$ | 371.45 | 207°C |

The compounds of the present invention exhibit valuable pharmacologic activities associated to a very low toxicity. Such characteristic are hereinbelow illustrated.

Acute toxicity

None of the test compounds caused lethal effects, both in short and long time, at the maximal tested doses, by oral administration in the rat (800 mg/kg) and by intraperitoneal administration in the mouse (400 mg/kg). The animals were kept under observation during a period of 10 days after the treatment.

As regards the intraperitoneal administration, a slight symptomatology of depressive kind was observed during the early 10-20 minutes, without showing ataxy, reduction of tonus nor body-righting reflexes, tremor, convulsive phenomena.

Pharmacologic activity

The activity tests were carried out on male Sprague-Dawley rats weighing 200±20 g and on male Swiss mice weighing 26±3 g.

The test compounds were administered orally by means of gastroesophageal tube, in the form of a suspension in a 10 ml/kg mucilage of 5% gum arabic; and intraperitoneally in the form of an aqueous suspension added with a few drops of Tween 80, in the constant volume of 10 ml/kg.

Antiinflammatory activity: carrageenin oedema in the rat

Screening for antiinflammatory activity was carried out by using the carrageenin oedema test

in the rat, based on procedure described by Winter et al. (Proc. Soc. Exp. Biol., 111, 544, 1962). The compounds under test and the control ones were administered orally one hour before the injection of 0.1 ml of 1% carrageenin in the right paw of the animals.

The volume of the paw was measured by means of a plethysmograph immediately before the injection and 2, 4 and 6 hours after.

The results were expressed as percentage inhibition of the oedema in the treated animals in comparison with the control animals, at the time of the maximum oedema development (fourth hour after the carrageenin injection).

The results are set out in Table 2.

All the tested compounds, at the oral dose of 100 ml/kg, exhibited an antiinflammatory activity; the amount of the anti-oedema effect found for the more active compounds, turned out on the whole higher, at equiponderant doses, than that of Acetylsalicylic acid (ASA); comparable to that of mephenamic acid (MFA) and slightly lower than that of Indomethacin (INDO). The more active compounds of the series, in decreasing order, were:

HA-36 > HA-32 > HA-34 > HA-38 > HA-37 > HA-33.

TABLE 2

ANTIINFLAMMATORY ACTIVITY - Oedema by carrageenin in the rat

| TREATMENT os | DOSE mg/kg | N° OF ANIMALS | OEDEMA INHIBITION % at the fourth hour | P |
|---|---|---|---|---|
| HA 30 | 100 | 8 | 35.6 ± 0.74 | <0.05 |
| HA 31 | 100 | 8 | 36.2 ± 0.43 | <0.05 |
| HA 32 | 100 | 8 | 49.6 ± 0.82 | <0.01 |
| HA 33 | 100 | 8 | 39.6 ± 0.76 | <0.05 |
| HA 34 | 100 | 8 | 48.8 ± 0.91 | <0.05 |
| HA 35 | 100 | 8 | 39.0 ± 0.68 | <0.01 |
| HA 36 | 100 | 8 | 52.7 ± 0.61 | <0.01 |
| HA 37 | 100 | 8 | 46.4 ± 0.78 | <0.01 |
| HA 38 | 100 | 8 | 46.6 ± 0.84 | <0.01 |
| HA 39 | 100 | 8 | 38.2 ± 0.68 | <0.05 |
| MFA | 100 | 8 | 49.2 ± 0.86 | <0.01 |
| ASA | 100 | 8 | 31.4 ± 0.37 | <0.05 |
| INDO | 10 | 8 | 57.3 ± 0.85 | <0.01 |

Analgesic activity

a) Tail finch test

The central analgesic activity of the compound according to the invention was assessed by the "tail finch" test as described by Bianchi et al., Brit. J. Pharmacol. 9, 220, 1954. This method is based upon the administration of the compounds by the intraperitoneal route and the estimate of the percentage of animals which stood for 10 seconds an hemostatic clip placed at the base of the finch, without turning out.

Those animals which during preliminary tests showed a normal reaction not higher than 4 seconds were employed for this test. The test was carried out 15, 30, 60 and 120 minutes after the pharmacologic treatment.

None of the compounds under test showed to possess central analgesic activity,up to doses of 50 mg/kg i.p.

b) Writhing test in rat

The analgesic activity of the compounds of the present invention was measured by the "writhing test" as described by Siegmund et al.(Soc. Exp. Biol. Med., 97, 729, 1957). The test compounds were administered orally, by gastric tube, 1 hour before intraperitoneal injection of phenylbenzoquinone (0.25 ml/rat of a 5% ethanol 0.025% solution). Five minutes after the injection, the number of writhes exhibited by each rat was recorded, until the 15th minute.

The obtained results are summarized in Table 3. All the test compound exhibit a marked analgesic action at oral doses of 5 and 0.5 mg/kg.

Compound HA 34 is the more active of the tested compounds. The analgesic action was lower than that of Indomethacin, but by far higher than that of Acetylsalicylic acid.

Comparison with mephenamic acid revealed the marked superiority of the tested compounds, with the exception of compound HA-30 which was substantially comparable to the reference compound.

TABLE 3

ANALGESIC ACTIVITY - Writhing test induced by phenyl-benzoquinone in the rat.

| TREATMENT os | DOSE mg/kg | N° OF ANIMALS | N° OF WRITHINGS in 10 MINUTES | % INHIBI-TION | P |
|---|---|---|---|---|---|
| Controls | --- | 20 | 55.1 ± 1.45 | -- | --- |
| HA 30 | 5<br>0.5 | 10<br>10 | 35.3 ± 1.64<br>31.8 ± 1.98 | 36<br>15 | <0.001<br><0.02 |
| HA 31 | 5<br>0.5 | 10<br>10 | 29.7 ± 1.76<br>43.5 ± 1.78 | 46<br>21 | <0.001<br><0.001 |
| HA 32 | 5<br>0.5 | 10<br>10 | 13.3 ± 2.06<br>28.6 ± 1.03 | 76<br>48 | <0.001<br><0.001 |
| HA 33 | 5<br>0.5 | 10<br>10 | 16.5 ± 1.43<br>40.2 ± 2.14 | 70<br>27 | <0.001<br><0.001 |
| HA 34 | 5<br>0.5 | 10<br>10 | 10.5 ± 1.72<br>41.9 ± 1.68 | 81<br>24 | <0.001<br><0.001 |
| HA 35 | 5<br>0.5 | 10<br>10 | 35.0 ± 1.42<br>44.6 ± 2.05 | 66<br>19 | <0.001<br><0.02 |
| HA 36 | 5<br>0.5 | 10<br>10 | 22.0 ± 1.14<br>43.0 ± 1.80 | 60<br>22 | <0.001<br><0.001 |
| HA 37 | 5<br>0.5 | 10<br>10 | 28.6 ± 1.26<br>45.2 ± 1.38 | 48<br>18 | <0.001<br><0.01 |
| HA 38 | 10<br>5 | 10<br>10 | 26.4 ± 1.12<br>44.1 ± 1.24 | 52<br>20 | <0.001<br>--- |
| HA 39 | 10<br>5 | 10<br>10 | 12.7 ± 2.01<br>43.0 ± 2.12 | 77<br>22 | <0.001<br><0.01 |
| MFA | 10<br>5 | 10<br>10 | 36.8 ± 2.24<br>41.9 ± 2.88 | 33<br>24 | < 0.001<br>< 0.001 |
| ASA | 50<br>5 | 10<br>10 | 33.0 ± 1.72<br>52.8 ± 1.46 | 40<br>++ | 0.001<br>--- |
| INDO | 5<br>0.5 | 10<br>10 | 6.9 ± 0.75<br>28.0 ± 1.45 | 87<br>49 | <0.001<br><0.001 |

Anti-exudative activity (acetic acid peritonitis in the rat)

The test was carried out according to the procedure described by Arrigoni-Martelli (Boll.Chim. Far. 107, 29, 1968). This method is based upon the evaluation of the anti-exudative effect of a drug at the peritoneal level, following a intraperitoneal injection of 10 ml/kg of a 0.5% acetic acid solution.

The compounds under test were administered orally one hour before the inflammatory agent; 30 minutes after the treatment the animals were killed and the fluid from the peritoneal cavity was drawn and measured.

The obtained results are reported in Table 4.

At the oral dose of 10 mg/kg the HA-40 and HA-31 derivatives, as well as acetylsalicylic and mephenamic acids, did not display valuable anti-exudating properties.

On the contrary, all the other tested compounds exhibited a marked anti-exudative activity, comparable or stronger than that of indomethacin at the oral dose of 1 mg/kg.

TABLE 4

ANTI-EXUDATIVE ACTIVITY - Acetic acid peritonitis in the rat

| TREATMENT os | DOSE mg/kg | N° OF ANIMALS | PERITONEAL EXUDATE ml ± E.S. | % INHIBITION | P |
|---|---|---|---|---|---|
| Controls | -- | 15 | 2.91 ± 0.12 | -- | -- |
| HA 30 | 10 | 5 | 2.50 ± 0.10 | 14 | n.s. |
| HA 31 | 10 | 5 | 2.47 ± 0.12 | 15 | 0.001 |
| HA 32 | 10 | 5 | 0.93 ± 0.14 | 74 | 0.001 |
| HA 33 | 10 | 5 | 1.04 ± 0.16 | 68 | 0.001 |
| HA 34 | 10 | 5 | 0.75 ± 0.11 | 64 | 0.001 |
| HA 35 | 10 | 5 | 1.28 ± 0.13 | 56 | 0.001 |
| HA 36 | 10 | 5 | 1.48 ± 0.10 | 49 | 0.001 |
| HA 37 | 10 | 5 | 1.10 ± 0.12 | 62 | 0.001 |
| HA 38 | 10 | 5 | 1.34 ± 0.10 | 54 | 0.001 |
| HA 39 | 10 | 5 | 1.39 ± 0.14 | 52 | 0.001 |
| MFA | 10 | 5 | 2.60 ± 0.15 | 6 | n.s. |
| ASA | 20 | 5 | 2.76 ± 0.13 | -- | -- |
| INDO | 10 | 5 | 0.18 ± 0.02 | 93 | 0.001 |
| | 1 | 5 | 0.70 ± 0.10 | 74 | 0.001 |

Ulcerogenic effect

Screening to determine the extent of gastric ulceration following oral administration of 400 mg/kg only of the compounds which showed to be the most active in the writhing test, was carried out accor ding to the procedure described by Bonfils et al. (Comp. Rend., 148, 881, 1954).

To rats fasted for 18 hours, with water ad libitum, were administered the compounds under test at "O" time and after 2 hours. Control rats were treated with established anti-inflammatory agents.

6 Hours after the beginning of the test, the animals were killed by ether inhalation.

Gastric ulceration was assessed on the ground of values ranging from O to 4:

O = no ulceration;

1 = slight iperhaemia and/or a few ulcers;

2 = severe iperhaemia and many punctiform ulcers;

3 = severe submucosal hemorrage, one large ulcer and many punctiform ulcers;

4 = severe submucosal hemorrage and and many large ulcers.

The ulcerogenic index was measured by the following formula (Lwoff. J.M., J. Pharmacol., 2, 81, 1971):

ulcerogenic index =

$$\frac{\text{ulceration degree x n° of animals showing ulceration}}{\text{n° of treated animals}} \text{ x 100.}$$

The tested compounds produced no ulceration in the rat, after administration of 400 mg/kg by oral route.

As a consequence of the absence of ulcerating effect and of letal effects after oral doses of 800 mg/kg, the compounds of the invention clearly demonstrate to possess therapeutic index and gastric tolerability substantially higher than the ones of the control drugs.

The results are set out in Table 5.

TABLE 5

ULCEROGENIC EFFECT IN THE RAT

| TREATMENT os | DOSE mg/kg | N° OF ANIMALS | % ANIMALS WITH ULCERS | ULCERATION DEGREE | ULCERATION INDEX |
|---|---|---|---|---|---|
| HA-32 | 400 | 8 | O | O | O |
| HA-33 | 400 | 8 | O | O | O |
| HA-34 | 400 | 8 | O | O | O |
| HA-35 | 400 | 8 | O | O | O |
| HA-36 | 400 | 8 | O | O | O |
| MFA | 200 | 8 | 50 | 1.0 | 50 |
| ASA | 200 | 8 | 78 | 1.5 | 112 |
| INDO | 20 | 8 | 100 | 2.5 | 250 |

Analgesic activity: $ED_{50}$ in the phenylbenzoquinone writhing test in the mouse.

The analgesic activity of compound HA-34 was measured according to the phenylbenzoquinone writhing

test in the mouse, as described by Siegmund et al. (Proc. Soc. Exp. Biol. Med. 97, 729, 1957), in order to determine:

a) $ED_{50}$ by oral administration of a test compound, 1 hour before the intraperitoneal injection of the inflammatory agent;

b) the duration of the analgesic effect, evaluating the degree of the protective effect on the peritoneal pain syndrome induced by oral administration of 1 mg/kg of the inflammatory agent, effected at various intervals of time.

The analgesic effect was determined by the number of writhes of the abdomen recorded during 10 minutes (from 5 to 15 minutes after the phenylbenzoquinone injection). Indomethacin and Piroxicam were used as the control drugs.

The results are listed in Tables 6 and 7.

HA-34 administeredl orally 1 hour before the test, has an $ED_{50}$ of 1 mg/kg. Under the same experimental conditions, Indomethacin and Piroxicam exhibit $ED_{50}$ of 0.5 mg/kg and 1.2 mg/kg, respectively. The duration of action of HA-34 is longer lasting than that of Indomethacin and substantially comparable with that of Piroxicam.

TABLE 6

WRITHING TEST BY PHENYLBENZOQUINONE IN THE MOUSE

Oral administration of the test compounds one hour before the test.

| TREATMENT | DOSE mg/kg | N° OF ANIMALS | N° OF WRITHES IN 10' ml ± E.S. | % INHIBITION | $ED_{50}$ mg/kg |
|---|---|---|---|---|---|
| Controls | -- | 10 | 52.4 ± 1.69 | -- | -- |
| HA-34 | 0.1 | 10 | 44.1 ± 1.12 | 15.8 | 1.0 |
| | 0.5 | 10 | 36.0 ± 1.33 | 31.3 | |
| | 1 | 10 | 17.2 ± 0.87 | 67.2 | |
| | 2 | 10 | 13.8 ± 1.12 | 73.6 | |
| | 5 | 10 | 10.6 ± 1.06 | 79.8 | |
| Controls | -- | 10 | 52.4 ± 1.69 | -- | -- |
| INDO | 0.1 | 10 | 40.4 ± 2.46 | 22.9 | 0.5 |
| | 0.5 | 10 | 27.2 ± 1.48 | 48.1 | |
| | 1 | 10 | 6.8 ± 0.76 | 87.0 | |
| | 5 | 10 | 4.5 ± 0.65 | 91.4 | |
| Controls | -- | 10 | 52.4 ± 1.69 | -- | -- |
| PIROXICAM | 0.1 | 10 | 46.2 ± 1.76 | 11.8 | 1.2 |
| | 0.5 | 10 | 37.5 ± 1.26 | 28.4 | |
| | 1 | 10 | 23.7 ± 2.18 | 54.8 | |
| | 2 | 10 | 20.0 ± 0.79 | 61.8 | |
| | 5 | 10 | 9.3 ± 1.44 | 82.2 | |

TABLE 7

DURATION OF THE ANALGESIC EFFECT

Oral administration at different times before the injection of phenylbenzoquinone. Oral dose: 1 mg/kg. Determination of n° of writhes in 10 min. (5 to 15 min. after the administration).

| TREATMENT | N° OF ANIMALS | ADMINISTRATION TIME | N° OF WRITHES IN 10' ml ± E.S. | % INHIBITION |
|---|---|---|---|---|
| Controls (physiol. solut.) | 40 | -- | 53.2 ± 1.26 | -- |
| HA-34 | 10 | 30 min. | 19.2 ± 1.18 | 63.9 |
| | 10 | 1 h | 18.4 ± 1.07 | 65.4 |
| | 10 | 3 h | 16.0 ± 1.26 | 69.9 |
| | 10 | 4 h | 18.4 ± 1.74 | 65.4 |
| | 10 | 6 h | 20.2 ± 1.24 | 62.0 |
| | 10 | 8 h | 21.6 ± 1.32 | 59.4 |
| | 10 | 24 h | 22.2 ± 1.46 | 58.3 |
| | 10 | 32 h | 36.9 ± 2.01 | 30.6 |
| INDOMETHACIN | 10 | 30 min. | 7.7 ± 1.18 | 85.5 |
| | 10 | 1 h | 6.5 ± 0.86 | 87.8 |
| | 10 | 3 h | 6.2 ± 0.70 | 88.3 |
| | 10 | 4 h | 6.8 ± 0.64 | 87.2 |
| | 10 | 6 h | 10.4 ± 0.56 | 80.4 |
| | 10 | 8 h | 21.2 ± 1.26 | 60.3 |
| | 10 | 24 h | 24.8 ± 1.15 | 53.4 |
| | 10 | 32 h | 39.4 ± 1.36 | 25.9 |
| PIROXICAM | 10 | 30 min. | 24.2 ± 1.62 | 54.5 |
| | 10 | 1 h | 22.7 ± 2.18 | 57.3 |
| | 10 | 3 h | 17.2 ± 1.12 | 67.6 |
| | 10 | 4 h | 19.5 ± 1.02 | 63.3 |
| | 10 | 6 h | 18.6 ± 1.12 | 65.0 |
| | 10 | 8 h | 21.2 ± 0.72 | 60.1 |
| | 10 | 24 h | 22.6 ± 1.37 | 57.5 |
| | 10 | 32 h | 46.8 ± 1.87 | 12.0 |

Anti-exudative activity: $ED_{50}$ in acetic acid peritonitis in the rat.

The $ED_{50}$ of the anti-exudative activity of HA 34 in comparison with Indomethacin and Piroxicam was determined.

The test compounds were administered by oral route 1 hour before intraperitoneal injection of acetic acid (10 ml/kg of a 0.5% solution).

The anti-exudative activity was determined on the basis of the peritoneal exudate of the treated animals, in comparison with the controls.

The results are reported in Table 8.

HA-34 and Piroxicam exhibit a similar anti-exudative activity ($ED_{50}$ = 0.7 mg/kg), about 2-3 times lower than that of Indomethacin ($ED_{50}$ = 0.3 mg/kg).

TABLE 8

ACETIC ACID PERITONITIS IN THE RAT.

Anti-exudative activity of HA-34, Indomethacin (INDO) and Piroxicam by oral administration, 1 hour before the i.p. injection of acetic acid. Determination of the peritoneal exudate volume 30 min. after the acetic acid injection.

| TREATMENT | DOSE mg/kg | N° OF ANIMALS | EXUDATE VOLUME ml ± E.S. | % INHIBITION | $ED_{50}$ mg/kg |
|---|---|---|---|---|---|
| Controls | -- | 12 | 2.58 ± 0.18 | -- | -- |
| HA-34 | 0.1<br>0.5<br>1<br>10<br>20 | 6<br>6<br>6<br>6<br>6 | 1.89 ± 0.14<br>1.34 ± 0.07<br>1.20 ± 0.10<br>0.54 ± 0.08<br>0.15 ± 0.07 | 26.7<br>48.0<br>53.2<br>78.8<br>94.2 | 0.7 |
| INDO | 0.1<br>0.5<br>1<br>10 | 6<br>6<br>6<br>6 | 1.62 ± 0.09<br>1.21 ± 0.07<br>0.70 ± 0.09<br>0.13 ± 0.04 | 37.2<br>53.0<br>72.8<br>94.8 | 0.3 |
| Piroxicam | 0.1<br>0.5<br>1<br>10<br>20 | 6<br>6<br>6<br>6<br>6 | 1.94 ± 0.08<br>1.37 ± 0.07<br>1.26 ± 0.06<br>0.49 ± 0.04<br>0.12 ± 0.06 | 24.6<br>46.8<br>51.2<br>80.0<br>95.2 | 0.7 |

Carrageenin pleuritis in the rat

Compound HA-34 was tested for the protective activity against the exudative pleuritis reaction induced by carrageenin. HA-34 was administered 30 min. before the injection of carrageenin, and 6 hours after it. The experiment was carried out on male Sprague-Dawley rats, anestethized with ethyl urethane, which were subjected, in their right pleural cavity, to injection of 1.5 ml of air and then 0.1 ml of a 1% carrageenin solution, in sterile normal saline, according to the procedure by Van Armann et al. (J. Pharmac. Exp. Ther., 150, 328, 1965). Indomethacin and Piroxicam were employed as the control drugs, under the same conditions.

18 Hours after the intrapleuric carrageenin injection, the animals were killed by means of ether inhalation, and the pleuric exudate was collected and measured.

The results are reported in Table 9.

HA-34, at the tested doses, exhibits a significant inhibiting action on the pleuric exudative reaction induced by carrageenin.

Such action is comparable to that of Piroxicam and lower than that of Indomethacin.

===

===

===

===

===

===

===

TABLE 9

ANTI-EXUDATIVE ACTIVITY

Carrageenin pleuritis in the rat

| TREATMENT | DOSE mg/kg | N° OF ANIMALS | EXUDATE VOLUME | % INHIBITION |
|---|---|---|---|---|
| Controls | -- | 10 | 2.76 ± 0.38 | -- |
| HA-34 | 5 | 5 | 1.80 ± 0.29 | 34.8 |
| | 10 | 5 | 1.02 ± 0.26 | 63.0 |
| INDO | 5 | 5 | 1.33 ± 0.34 | 51.8 |
| | 10 | 5 | 0.76 ± 0.20 | 72.4 |
| PIROXICAM | 5 | 5 | 1.67 ± 0.33 | 39.5 |
| | 10 | 5 | 0.99 ± 0.40 | 64.1 |

CLAIMS

1. 1,3,4-Thiadiazol-/3,2-a/-thieno-/2,3-d/-pyrimidin-5-/H/one derivatives of formula (I)

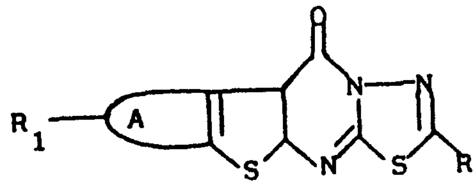

(I)

wherein:

R is H, $CH_3$, $C_2H_5$, $SCH_3$, $SC_2H_5$, $OC_2H_5$, $C_6H_5$, $C_6H_4F(p)$, $C_6H_4OC_2H_5(o)$;

A is tetramethylene, trimethylene or a -CH=CH-CH=CH group,

$R_1$ is H, $C_1$-$C_4$ alkyl, $C_1$-$C_4$ alcoxy, hydroxy.

2. Compound according to claim 1, wherein A = trimethylene, R = methyl and $R_1$ = hydrogen.

3. Compound according to claim 1, wherein A = -CH=CH-CH=CH, R = methyl and $R_1$ = 6-ethyl.

4. Compound according to claim 1, wherein A = tetramethylene, R = ethyl and $R_1$ = hydrogen.

5. Compound according to claim 1, wherein A = tetramethylene, R = phenyl and $R_1$ = hydrogen.

6. Compound according to claim 1, wherein A = tetramethylene, R = methylmercapto and $R_1$ = hydrogen.

7. Compound according to claim 1, wherein A = -CH=CH-CH=CH-, R = ethylmercapto and $R_1$ = 6-ethyl.

8. Compound according to claim 1, wherein A = tetramethylene, R = o-ethoxyphenyl and $R_1$ = hydrogen.

9. Compound according to claim 1, wherein A = trimethylene, R = ethoxy, and $R_1$ = 6-ethoxy.

10. Compound according to claim 1, wherein A = tetramethylene, R = p-fluoro-phenyl and $R_1$ = 6-ethoxy.

11. Compound according to claim 1, wherein A = trimethylene, R = o-ethoxyphenyl and $R_1$ = 6-hydroxy.

12. Pharmaceutical composition having analgesic and/or antiinflammatory activity, containing one or more compounds according to claims 1-11 as the active principle.

CLAIMS

1. Process for the preparation of compounds of formula I

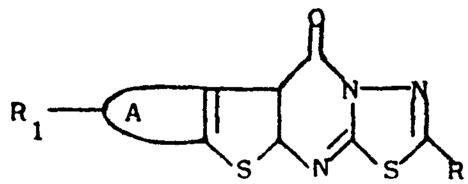

(I)

wherein:

R is H, $CH_3$, $C_2H_5$, $SCH_3$, $SC_2H_5$, $OC_2H_5$, $C_6H_5$, $C_6H_4F(p)$, $C_6H_4OC_2H_5(o)$;

A is tetramethylene, trimethylene or a -CH=CH-CH=CH group,

$R_1$ is H, $C_1$-$C_4$ alkyl, $C_1$-$C_4$ alcoxy, hydroxy, characterized by reacting 2-amino-3-carbalcoxy thiophenes of formula II

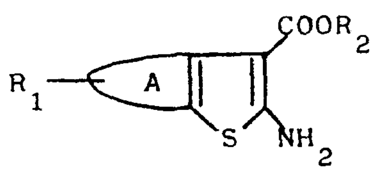

(II)

wherein R has the above mentioned meanings and X is an halogen.

2. Process according to claim 1, characterized by operating in the absence of solvents at temperatures ranging from 80 to 220°C.